# FASCICULE DE BREVET EUROPEEN

(11) **EP 1 027 106 B1**
(45) Date de publication et mention de la délivrance du brevet: **25.02.2009**
(21) Numéro de dépôt: 98952445.9
(22) Date de dépôt: 30.10.1998
(51) Int. Cl.: A61N 5/06

(54) **DISPOSITIF DE PHOTOTHERAPIE MULTIDIRECTIONNELLE INTENSIVE**
VORRICHTUNG ZUR MULTIDIREKTIONELLEN INTENSIVEN PHOTOTHERAPIE
INTENSIVE MULTIDIRECTIONAL PHOTOTHERAPY DEVICE

(30) Priorité: 30.10.1997 BE 9700872
(43) Date de publication de la demande: 16.08.2000
(60) Demande divisionnaire: 07102073.9
(73) Titulaire: Medestime S.A., 6001 Marcinelle (BE)
(72) Inventeur: Gysens, Lucien, B-6032 Charleroi (BE); Wauters, Marie Yvonne, B-6032 Charleroi (BE)
(74) Mandataire: Van Malderen, Joëlle
(86) Numéro de dépôt international: PCT/BE1998/000164
(87) Numéro de publication internationale: WO 1999/022813

(56) Documents cités:
- DE-A- 3 910 238
- FR-A- 2 193 628
- FR-A- 2 648 036
- GB-A- 2 216 012
- US-A- 3 705 576
- JOSEPH W. SAUSVILLE: "Blue Lamps in Phototherapy of Hhyperbilirubinemia" JOURNAL OF THE ILLUMINATION ENGINEERING SOCIETY, 17 - 20 août 1971, pages 112-118, XP002072623 PHILADELPHIA
- LETTRE TRIMESTRIELLE DE L'ASSOCIATION PHOTOTHÉRAPIE: PHOSPHOROS, vol. 3, avril 1989 (1989-04), - 3006
- CALDERA, SENDER: "Indications et technique de la photothérapie intensive chez le nouveau-né à terme" ARCH. PEDIATR., vol. 4, août 1997 (1997-08), pages 803-804,
- "LETTRE TRIMESTRIELLE DE L'ASSOCIATION PHOTOTHERAPIE" PHOSPHOROS, vol. 3, avril 1989 (1989-04),
- LETTRE TRIMESTRIELLE DE L'ASSOCIATION PHOTOTHÉRAPIE: PHOSPHOROS, vol. 3, avril 1989 (1989-04),

## Description

### Objet de l'invention

La présente invention se rapporte à un dispositif de photothérapie multidirectionnelle dite intensive, destiné à traiter un nouveau-né prématuré ou à terme souffrant ou susceptible de développer un ictère grave.

### Arrière-plan technologique à la base de l'invention

Il y a de plus en plus de bébés prématurés déclarant un ictère grave nécessitant une exsanguino-transfusion. L'ictère pouvant se développer rapidement et de façon exponentielle, il faut intervenir très rapidement si on veut éviter des dégâts neurologiques irréversibles. Ces prématurés doivent recevoir des soins lourds en couveuse et cela sans interruption.

Jusqu'à présent, le traitement de l'ictère consistait en une exsanguino-transfusion permettant de dissocier la bilirubine formant un poison avant la transfusion du nouveau-né.

Etant donné que la plupart des hyperbilirubinémies sont dues aux incompatibilités sanguines parentales (incompatibilité des facteurs rhésus, A, B, O, etc.), il est impossible de prélever du sang aux parents pour le transfuser au nouveau-né.

### Etat de la technique

L'ictérie natale est également traitée depuis de nombreuses années dans les cliniques pédiatriques par la photothérapie de la peau des nouveau-nés.

Le document GB-A-2216012 décrit un appareillage comprenant une incubatrice réalisée en un matériau transparent, munie à sa partie supérieure d'un complexe de tubes fluorescents disposés parallèlement les uns aux autres sous une surface réfléchissante, afin d'illuminer depuis le haut le nouveau-né qui se trouve à l'intérieur de l'incubatrice.

Un support comprenant, incorporée, une telle surface réfléchissante est également décrit dans la demande de brevet DE-A-3910238. Ce type d'appareillage et de support oblige le personnel de service à faire tourner fréquemment le nouveau-né afin d'irradier sa peau le plus uniformément possible de tous les côtés. Ceci implique un accroissement de la durée de la thérapie, un certain désagrément pour le nouveau-né avec la difficulté d'obtenir un traitement vraiment uniforme sur toutes les parties de sa peau.

Le document FR-A-2193628 décrit un appareillage de photothérapie particulièrement complexe et encombrant, comprenant un habitacle entièrement réalisé en un matériau transparent, comportant une couchette sur laquelle est étendu le nouveau-né et deux groupes de sources lumineuses disposés respectivement au-dessus et au-dessous de l'habitacle. Cependant, un tel appareillage nécessite l'utilisation de tubes à lumière de grande taille et de grande consommation énergétique, étant donné que le nouveau-né traité est placé beaucoup trop loin des sources de lumière. De plus, le rayonnement obtenu par un tel dispositif (de l'ordre de 1 mW/cm² de peau du patient ont des dimensions trop importantes (pouvant aller jusqu'à une longueur de 520 mm pour un diamètre de 30 mm) pour créer un dispositif se longeant dans des incubateurs. Ils nécessitent l'emploi de ballasts et de broutons de marche/arrêt pour leur allumage, susceptibles de provoquer la formation d'une étincelle à chaque démarrage. Ceci est particulièrement dangereux lors d'un traitement en incubateur, qui nécessite souvent des pourcentages d'oxygène dépassant 21% et pouvant même aller jusqu'à 40 et 60%. Il en résulte que les étincelles générées par ces appareils excluent d'office cette pratique.

Le souhait des spécialistes est de disposer d'un système qui permettre de soigner les enfants en détresse grave devant à la fois subir des soins lourds en incubateur, en couveuse fermée, et une phytothérapie intensive efficace et adaptable en fonction de la gravité de l'ictère qu'ils développent, tout en maintenant une surveillance adéquate du nouveau-né traité.

### Buts de l'invention

La présente invention vise à fournir un nouveau dispositif ou appareillage de photothérapie qui ne présente pas les inconvénients des dispositifs de l'état de la technique et qui soit de conception simple et modulable, de manière à pouvoir être utilisé pour traiter en sécurité un nouveau-né susceptible de développer ou atteint d'une ictérie, en particulier en incubateur ou en couveuse fermée, de manière à limiter ou éviter tout traumatisme au nouveau-né, en particulier des opérations d'exsanguino-transfusion.

### Eléments caractéristiques de l'invention

La présente invention concerne un dispositif de photothérapie destinée au traitement de l'ictère d'un patient nouveau-né qui comporte :
- un bac à lumière comprenant au moins une source de rayonnement lumineux agencée dans le bac pour éclairer ledit patient, le bac à lumière étant essentiellement hermétique incorporant des ventilateurs ainsi que des moyens d'aspiration et d'évacuation d'air.
- un support pour l'étalement du dit patient, ledit support étant au moins partiellement mais essentiellement transparent ou teinté de façon à laisser passer le rayonnement lumineux provenant du bac à lumière, le support étant disposé au-dessus du bac à lumière de manière à exposer le patient maintenu sur le support par le dessous en référence à son sens de gravité, permettant ainsi de mieux exposer les zones à forte concentration de bilirubine ayant tendance à suivre la gravité,
- ladite au moins une source de rayonnement lumineux comportant des lampes et le bac à lumière comportant un dispositif de contrôle de la température,
caractérisé en ce que les lampes ont un spectre de lumière d'une longueur d'ondes allant de 420 à 650 nm avec une présence importante de lumière bleue et verte et permet de fournir une puissance d'éclairement énergétique supérieur à 3 mW par cm² de peau du patient, de préférence supérieur à 5 mW par cm² de peau du patient et en ce que le dispositif de contrôle de la température enclenche un thermostat commandant l'allumage ou l'extinction des lampes et l'activation des dits ventilateurs.

Avantageusement, le support pour l'étalement du patient et réalisé en un matériau filtrant ou bloquant le passage des rayonnements ultra violets et infrarouges.

De préférence, le support pour l'étalement dudit patient comprend un matelas de préférence un matelas de type gel. De manière avantageuse le support pour l'étalement du patient comprend ou consiste en une plaque de plexiglas®

Selon une forme d'exécution préférée de l'invention, les lampes sont dépourvues d'allumeur.

Selon une autre forme d'exécution préférée de l'invention, les lampes sont agencées en alternance sur des côtés mutuellement opposés du bac à lumière.

Avantageusement, le dispositif de l'inventeur comprend une ou plusieurs coupoles agencées du côté opposé au bac à lumière par rapport au support et comporte au moins une des dites lampes.

Selon une forme d'exécution préférée de l'invention, le fond ou les parois latérales du bac à lumière ou des coupoles comportent un ou plusieurs éléments réfléchissant disposés de manière à assurer un éclairement du patient.

Un dernier aspect de l'invention concerne un incubateur ou une couveuse fermée incorporant le dispositif de photothérapie de l'invention.

### Description détaillée de l'invention

La présente invention concerne un dispositif de photothérapie, dite intensive, permettant de traiter un nouveau-né (prématuré ou à terme) souffrant d'ictère grave, en maternité, en chambre maternelle et même dans un milieu fermé tel qu'un incubateur ou une couveuse fermée, tout en limitant ou en évitant l'exsanguino-transfusion et en garantissant une sécurité optimale du nouveau-né traité.

Le dispositif de photothérapie de l'invention comporte (fixé avantageusement sur une même armature) un bac à lumière comprenant au moins une source de rayonnement lumineux (ladite source de rayonnement lumineux étant agencée dans le bac à lumière pour éclairer ledit patient) et un support pour l'étalement du patient, ledit support étant au moins partiellement (mais essentiellement) transparent ou teinté de façon à laisser passer le rayonnement lumineux provenant du bac à lumière.

Dans le dispositif de photothérapie de l'invention, le support est disposé au-dessus du bac à lumière de manière à exposer le patient (maintenu sur ce support par le dessous en référence au sens de gravité), permettant ainsi de mieux exposer les zones à forte concentration en bilirubine présentes dans le corps du patient et ayant tendance à suivre la gravité.

Selon l'invention, on entend par "bac à lumière" un ensemble hermétique susceptible de comporter une ou plusieurs sources émettrices d'un rayonnement lumineux, telles que des lampes tubes (ou éventuellement d'autres sources lumineuses raccordées à des fibres optiques) ainsi que tous les éléments permettant la modulation ou l'extinction de ces sources lumineuses. Le bac à lumière de l'invention incorpore également différents moyens assurant que tout échauffement excessif de fluides ou de gaz provoqué par ces sources émettrices de rayonnement lumineux n'affecte pas le patient à traiter et autorise uniquement l'émission de rayonnement lumineux (de préférence filtré, sans infrarouges et sans ultraviolets) vers le nouveau-né à traiter.

En outre, le bac à lumière comprend des prises électriques et des prises d'air extérieures permettant d'alimenter les différents éléments électriques, mécaniques ou électroniques du dispositif, en particulier du bac à lumière, tels que des ventilateurs, des cheminées d'amenée et d'évacuation d'air via des cônes d'aspiration, etc.

Le système de régulation thermique consiste en un dispositif d'évacuation d'air comportant des ventilateurs placés dans les coins du bac à lumière et autorisant une circulation d'air efficace vers des cônes d'aspiration et d'évacuation d'air.

L'armature maintenant le bac à lumière et le support selon une orientation permettant d'exposer le patient au rayonnement lumineux est adaptée de manière à permettre l'incorporation du dispositif de photothérapie dans un incubateur ou dans une couveuse fermée.

Pour une sécurité optimale, le bac à lumière est parfaitement étanche, et évite tout contact électrique ou mécanique avec des éléments placés dans l'incubateur ou la couveuse fermée. Une telle disposition évite toute formation d'une étincelle ou d'un choc pouvant avoir des conséquences graves pour le patient.

Selon l'invention, les sources de rayonnements lumineux sont conçues pour obtenir un rayonnement lumineux supérieur à 3 mW par cm² de peau, de préférence supérieur à 5 mW par cm² de peau du patient traité. Ces sources de rayonnement lumineux sont par exemple constituées de lampes à lumière bleue ou verte présentant de préférence un spectre de lumière bleue mélangée à la verte, adaptées à tout type de peau d'un patient, y compris les enfants noirs ou mulâtres.

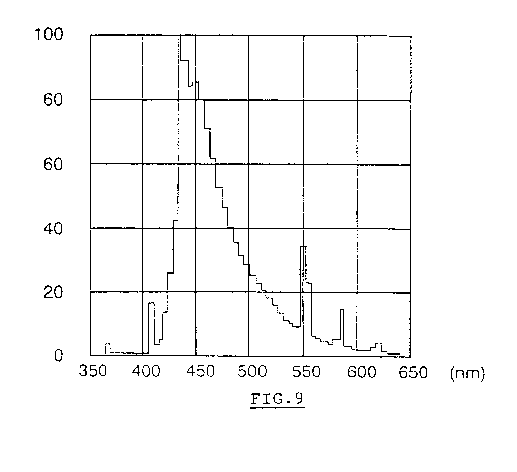

De préférence, on utilise des sources lumineuses constituées de lampes dont l'analyse spectrale révèle une lumière bleue et verte à trois bandes (de préférence d'une longueur d'ondes allant de 420 à 650 nm) et permettant de fournir une puissance d'éclairement énergétique plus importante que celle dans tubes classiques, avec une bande disponible plus large et une dissociation de la bilirubine plus rapide (en particulier des lampes tubes de type D.Z.C 11/75 à culot 2.G.7.4 à quatre broches avec une longueur d'ondes de 420 à 700 nm (telles que prévues selon le diagramme de la Commission Internationale d'Eclairage (code 1931)) (voir figure 9).

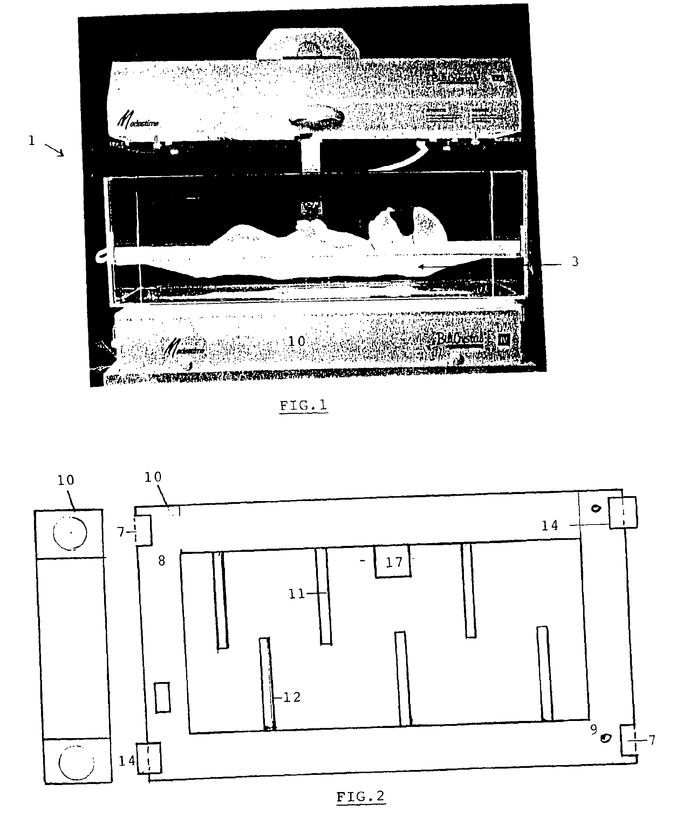

Ces lampes tubes de faible dimensions sont dépourvues d'allumeur, et sont aisément adaptables dans le bac à lumière de l'invention. Elles permettent leur disposition dans une géométrie (voir figure 2) assurant une émission lumineuse efficace sur le patient à traiter.

Le dispositif de photothérapie selon une forme d'exécution préférée de l'invention peut également comprendre une ou plusieurs coupoles incorporant également des sources de rayonnement lumineux, lesdites coupoles étant aptes à exposer le patient (reposant sur le support) par le dessus par référence au sens de gravité dudit patient et sont agencées du côté opposé au bac à lumière par rapport au support. Avantageusement, les parois des coupoles, du bac à lumière et éventuellement de l'armature, comportent des éléments réfléchissant le rayonnement lumineux émis par les sources vers le patient à traiter, de manière à assurer un traitement sur toutes la peau du patient.

En outre, les sources lumineuses disposées dans les coupoles ou dans le bac à lumière sont avantageusement agencées en alternance sur des côtés mutuellement opposés du bac à lumière ou de la coupole, de façon à permettre une émission de rayonnement lumineux croisée favorisant la qualité de l'éclairement du patient et permettant une circulation d'air optimale dans le bac à lumière ou dans la coupole, ce qui évite toute modification de température susceptible d'affecter le patient.

Le dispositif peut également comporter un ou plusieurs moyens de contrôle de l'émission de rayonnement lumineux en fonction de la répartition de la bilirubine dans le corps du patient à traiter, et/ou un dispositif ou une source de rayonnement lumineux additionnelle à hauteur de l'emplacement de la tête du patient, qui est une zone susceptible de comporter une concentration accrue en bilirubine.

Ce moyen de contrôle peut par exemple être un thermostat qui stoppe ou réduit automatiquement l'éclairage ou enclenche des ventilateurs permettant d'abaisser la température au sein du bac à lumière, de la coupole ou du milieu fermé où se trouve le nouveau-né traité. Ce système de thermostat peut être combiné avec un second moyen fixé directement sur le patient nouveau-né, tel qu'un thermomètre de précision relié à un thermostat électronique fixé directement sur le nouveau-né (sonde cutanée fixée sur l'abdomen du patient), qui contrôlera les variations de température du nouveau-né. Dans le cas d'une hyperthermie dépassant 39,5 °C, le nouveau-né à terme et surtout le nouveau-né prématuré, peut passer en convulsions. Le dispositif permettra d'enclencher différents mécanismes de prévention de telles complications ou permettra d'alerter le personnel médical ou infirmier (alarmes pouvant le cas échéant être reliées à une centrale de monitoring).

D'autres systèmes de contrôle des éléments intervenant dans le dispositif de l'invention ou du patient traité peuvent être adaptés par l'homme du métier en fonction des améliorations apportées à l'appareillage de traitement des nouveau-nés, ou en fonction des affections susceptibles d'affecter le nouveau-né.

Selon un mode préféré de l'invention, le support du dispositif de l'invention est réalisé en un matériau filtrant ou bloquant le passage des rayonnements ultraviolets et infrarouges.

Selon une forme d'exécution préférée de l'invention, le support comprend un matelas, de préférence un matelas de type gel, ayant une forme assurant un confort et une ergonomie optimaux. De préférence, ce matelas de type gel comporte une ou plusieurs rainures, de préférence selon une orientation quadrillé, favorisant l'écoulement des excréments du patient nouveau-né.

Selon une variante d'exécution, le support comprend ou consiste en un hamac dont les mailles laissent passer le rayonnement lumineux émis depuis le bac à lumière ou éventuellement réfléchi par les parois de l'armature ou d'une ou plusieurs coupoles.

Selon une variante d'exécution, le support peut comprendre ou consister en une plaque réalisée en un matériau tel que le plexiglas^{®}, permettant un filtrage (de préférence de type "écran total") des ultraviolets et des infrarouges.

Les autres parties du bac à lumière, des coupoles ou de l'armature peuvent être réalisées en une matière plastique classique telle que le polyuréthanne, obtenues par moulage ou injection à basse pression ou réalisées de façon monobloc. Ces différentes parties peuvent être réalisées en une seule pièce ou adaptées de façon modulable et ergonomique de manière à favoriser l'usage et l'entretien du dispositif de photothérapie de l'invention par le personnel technique ou infirmier.

Le bac à lumière peut également être recouvert par une plaque formant une jupe d'étanchéité périphérique pour ledit bac, dont le volume est adapté de manière à permettre une circulation d'air pulsé dans un incubateur ou une couveuse sans entrave. En particulier, la hauteur des parois de l'extrémité du bac à lumière ou de l'armature incorporant le bac à lumière correspond sensiblement à la hauteur de la tête d'un patient couché (une hauteur comprise entre 15 et 20 cm), de façon à ce que ladite paroi dépasse légèrement la tête du patient couché, forçant ainsi une circulation d'air au-dessus du patient sans que celui-ci n'y soit directement exposé.

Comme mentionné plus haut, le dispositif peut comporter une ou plusieurs coupoles éventuellement solidaires de l'armature du dispositif de photothérapie de l'invention ou pouvant être fixées sur un montant indépendant monté sur roues. Les coupoles avantageusement fixées sur un montant laissent un espace pour l'incorporation d'autres moyens d'alimentation ou de traitement du patient (baxter, système de chauffage, ventilateurs, etc.).

Les coupoles ou les montants supportant ces coupoles peuvent être avantageusement réglables en hauteur ou de manière angulaire, selon le type de traitement à apporter au patient.

D'autres particularités et caractéristiques de l'invention apparaîtront dans la description détaillée de l'invention et les exemples suivants en référence aux figures annexées.

### Brève description des figures

- La figure 1: représente une vue complète du dispositif de l'invention.
- Les figures 2 et 3: représentent des vues schématiques en plan des agencements particuliers du bac à lumière du dispositif de l'invention.
- Les figures 4 et 5: représentent des vues en perspective du bac à lumière de l'invention.
- La figure 6: représente des vues en perspective des coupoles du dispositif de l'invention.
- La figure 7: représente le montant supportant les coupoles et le support du dispositif de l'invention.
- La figure 8: représente le dispositif d'évacuation d'air du bac à lumière de l'invention.
- La figure 9: représente le diagramme d'émission de rayonnement lumineux utilisé dans le dispositif de l'invention.
- La figure 10: représente une synthèse des résultats obtenus par le dispositif de l'invention.

### Exemples

### Exemple 1

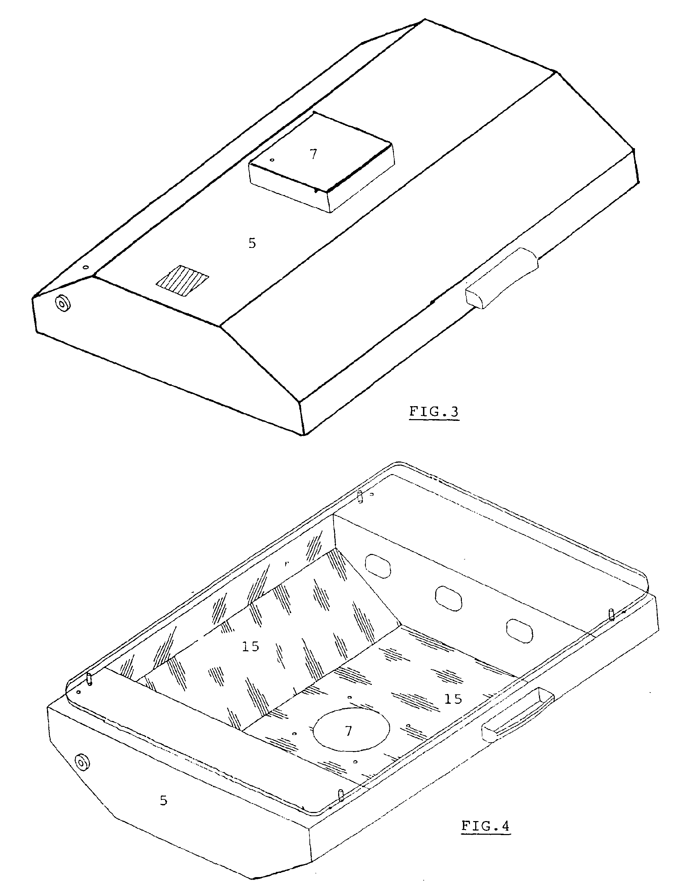

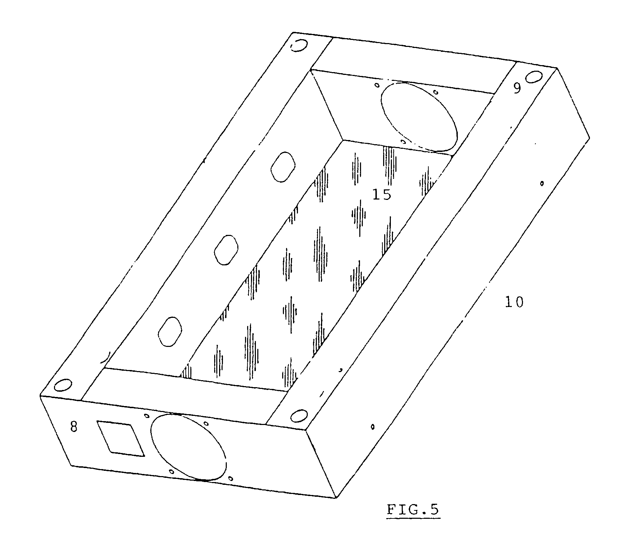

Le dispositif de photothérapie 1 tel que représenté dans les figures 1 à 8 comporte une armature 2 supportant un bac à lumière 10 et un support 3 pour le patient à traiter (qui consiste, dans la figure 1, en un hamac adapté pour le patient à traiter). Au-dessus du patient, l'armature 2 supporte également une coupole unique 5 éclairant par le dessus le nouveau-né.

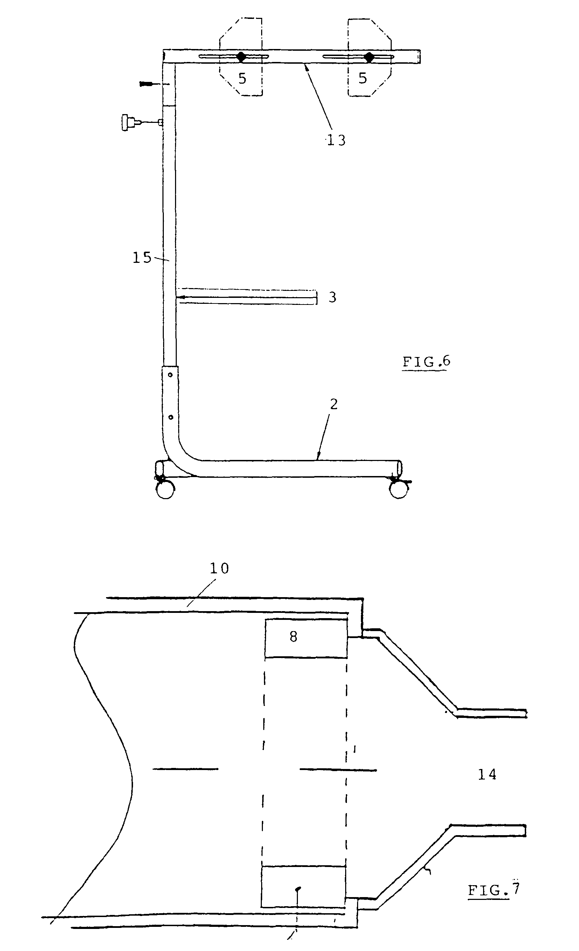

Le bac à lumière 10 tel que représenté dans les figures 2 à 5 comporte une succession de lampes 11, 12 agencées sur les côtés mutuellement opposés du bac à lumière 10. Dans les coins 8 et 9 du bac à lumière 10, on a incorporé des ventilateurs 7 permettant une circulation efficace d'air vers des cheminées d'évacuation 14, éventuellement via des cônes d'évacuation tels que représentés dans la figure 7.

Le bac à lumière 10 comporte également un dispositif de contrôle de la température, enclenchant un thermostat 17 commandant l'allumage ou l'extinction des lampes 11, 12 et l'activation des ventilateurs 7 précités.

Tel que représenté à la figure 6, le dispositif peut comporter plusieurs coupoles 5 réglables par une poignée et laisse entre lesdites coupoles 5 un espace 13 où peut être intégré un dispositif de chauffage, un baxter, etc.

Les coupoles 5 peuvent être maintenues par un montant 16 indépendant de l'armature 2 supportant le bac à lumière 10 et le support 3 ou être solidaires de ladite armature 2.

Le fond ou les parois latérales du bac à lumière 10 ou des coupoles 5 peuvent comporter un ou plusieurs éléments réfléchissants 15, disposés de manière à assurer un éclairement optimal du patient (de préférence toute la surface de la peau du patient doit être éclairée).

Les sources de rayonnement lumineux sont constituées de lampes tubes 11, 12 dont le spectre de rayonnement lumineux est représenté à la figure 9 (spectre de lumière bleue mélangée à la verte). Cependant, d'autres types de spectres adaptés à la peau d'un patient tel qu'un enfant noir ou un enfant mulâtre peuvent être utilisés, avec une présence plus importante de lumière verte.

### Exemple 2

Le dispositif de photothérapie de l'invention a été utilisé pour traiter différents nouveau-nés souffrant d'ictère grave, et permet un éclairement énergétique de lumière, en particulier de la composante bleue délivrée par les lampes tubes, à des valeurs supérieures à 2 mW par cm², de préférence à des valeurs supérieures à 4 mW par cm² de peau. Les nouveau-nés traités sont maintenus sous surveillance par les moyens de monitorage de l'invention et sous surveillance médicale, qui garantissent que l'enfant est exposé correctement au rayonnement lumineux.

Le dispositif de l'invention permet de remplir les conditions de la circulaire BH n° 21 du Ministère Français des Affaires Sociales et de la Santé et de la Ville, car il répond aux paramètres principaux requis, à savoir une source lumineuse adéquate, une distance de la source à l'enfant adéquate, un traitement de la surface cutanée complète de l'enfant sous réserve qu'aucune lésion rétinienne n'affecte le nouveau-né traité, un centrage adéquat de l'enfant sous la source lumineuse, un appareillage modulable, à haute performance et à maintenance aisée.

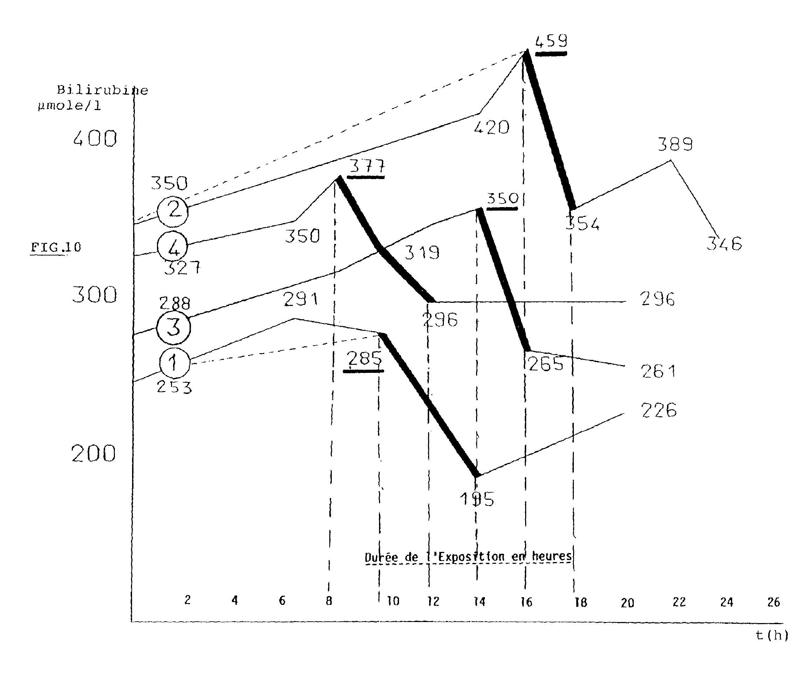

La figure 10 montre que 4 patients traités par le dispositif de l'invention présentaient, après une courte durée d'exposition, une chute de leur concentration en bilirubine (trait noir marqué). Ensuite, les patients étaient traités selon une photothérapie classique. Le court laps de temps pendant lequel les enfants sont traités par le procédé de photothérapie de l'invention est suffisant pour permettre une chute significative de la bilirubine (de 23 à 30%) et pour sortir de la zone dangereuse. Suite à ce traitement, on observe soit un taux de bilirubine stationnaire, soit une nouvelle ascension de ce taux au moment du passage à une photothérapie classique, témoignant de la différence d'efficacité de ces deux types de photothérapie.

## Revendications

1. Dispositif (1) de photothérapie destiné au traitement de l'ictère d'un patient nouveau-né, qui comporte ;
- un bac à lumière (10) comprenant au moins une source de rayonnement lumineux agencée dans le bac pour éclairer ledit patient, le bac à lumière (10) étant un ensemble hermétique incorporant des ventilateurs (7) placés dans les coins du bac à lumière (10) ainsi que des moyens d'aspiration et d'évacuation d'air (14) ;
- un support (3) pour l'étalement du dit patient, ledit support étant au moins partiellement, mais essentiellement, transparent ou teinté de façon à laisser passer le rayonnement lumineux provenant du bac à lumière (10), le support (3) étant disposé au-dessus du bac à lumière (10), de manière à exposer le patient maintenu sur le support (3) par le dessous en référence à son sens de gravité, permettant ainsi de mieux exposer les zones à forte concentration de bilirubine ayant tendance à suivre la gravité ; et dans lequel dispositif ladite au moins une source de rayonnement lumineux comporte des lampes (11, 12) et le bac à lumière (10) comporte un dispositif de contrôle de la température, **caractérisé en ce que**
- les lampes ont un spectre de lumière d'une longueur d'ondes allant de 420 à 650 nm avec une présence importante de lumière bleue et verte et permet de fournir une puissance d'éclairement énergétique supérieur à 3mW par cm² de peau du patient ;
- et le dispositif de contrôle de la température enclenche un thermostat (17) commandant l'allumage ou l'extinction des lampes (11, 12) et l'activation des dits ventilateurs (7).

2. Dispositif selon la revendication 1, **caractérisé en ce que** l'éclairement énergétique du patient obtenu par le rayonnement lumineux est supérieur à 5mW par cm2 de peau du patient.

3. Dispositif selon l'une quelconque des revendications 1 ou 2, **caractérisé en ce que** le support (3) est réalisé en un matériau filtrant ou bloquant le passage des rayonnements ultraviolet et infrarouge.

4. Dispositif selon l'une quelconque des revendications précédentes, **caractérisé en ce que** le support (3) comprend un matelas.

5. Dispositif selon la revendication 4 dans lequel le matelas est un matelas de type gel.

6. Dispositif selon l'une quelconque des revendications précédentes, **caractérisé en ce que** le support (3) comprend ou consiste en une plaque en plexiglas®.

7. Dispositif selon la revendication 1, **caractérisé en ce que** les lampes sont dépourvues d'allumeur.

8. Dispositif selon l'une quelconque des revendications précédentes, **caractérisé en ce que** les lampes (11, 12) sont agencées en alternance sur des côtés mutuellement opposés du bac à lumière (10).

9. Dispositif selon l'une quelconque des revendications précédentes, **caractérisé en ce qu'**il comprend une ou plusieurs coupoles (5) agencées du côté opposé au bac à lumière (10) par rapport au support (3) et comportant au moins une des dites lampes.

10. Dispositif selon l'une quelconque des revendications précédentes, **caractérisé en ce que** le fond ou les parois latérales du bac à lumière (10) ou des coupoles (5) comportent un ou plusieurs éléments réfléchissants disposés de manière à assurer un éclairement du patient.

11. incubateur ou couveuse fermée incorporant le dispositif de photothérapie selon l'une quelconque des revendications précédentes.

## Claims

1. A phototherapy device (1) intended for treating jaundice of a newborn patient, which includes:
- a light trough (10) comprising at least one source of light radiation laid out in the trough in order to illuminate said patient, the light trough (10) being a leak-proof assembly incorporating fans (7) placed in the corners of the light trough (10) as well as air suction and discharge means (14);
- a support (3) for spreading out said patient, said support being at least partly, but essentially transparent or tinted so as to let through the light radiation from the light trough (10), the support (3) being positioned above the light trough (10), so as to expose the patient held on the support (3) from under with reference to its direction of gravity, thereby allowing better exposure of the areas with a strong bilirubin concentration tending to follow gravity; and device in which said at least one source of light radiation includes lamps (11, 12) and the light trough (10) includes a device for controlling temperature, **characterized in that**
- the lamps have a light spectrum with a wavelength ranging from 420 to 650 nm with significant presence of blue and green light and by which an energetic illumination power above 3 mW per cm² of patient's skin may be provided;
- and the temperature control device triggers a thermostat (17) controlling the ignition or extinction of the lamps (11, 12) and the activation of said fans (7).

2. The device according to claim 1, **characterized in that** the energetic illumination of the patient obtained by the light radiation is above 5 mW per cm² of patient's skin.

3. The device according to any of claims 1 or 2, **characterized in that** the support (3) is made in a filtering material or a material blocking the passage of ultraviolet and infrared radiations.

4. The device according to any of the preceding claims, **characterized in that** the support (3) comprises a mattress.

5. The device according to claim 4, wherein the mattress is a mattress of the gel type.

6. The device according to any of the preceding claims, **characterized in that** the support (3) comprises or consists of Plexiglas®.

7. The device according to claim 1, **characterized in that** the lamps are without any igniter.

8. The device according to any of the preceding claims, **characterized in that** the lamps (11, 12) are alternately laid out on sides opposite to each other of the light trough (10).

9. The device according to any of the preceding claims, **characterized in that** it comprises one or several cupolas (5) laid out on the side opposite to the light trough (10) relatively to the support (3) and including at least one of said lamps.

10. The device according to any of the preceding claims, **characterized in that** the bottom or the sidewalls of the light trough (10) or of the cupolas (5) include one or several reflecting members positioned so as to provide illumination of the patient.

11. A closed incubator or brooder incorporating the phototherapy device according to any of the preceding claims.

## Patentansprüche

1. Lichttherapievorrichtung (1) zur Behandlung von Ikterus bei einem neugeborenen Patienten, die Folgendes umfasst:
- eine Lichtkammer (10), die mindestens eine Lichtstrahlungsquelle umfasst, die in der Kammer angeordnet ist, um den Patienten zu beleuchten, wobei die Lichtkammer (10) eine hermetische Einheit ist, in der Ventilatoren (7), die in den Ecken der Lichtkammer (10) angeordnet sind, sowie Luftansaug- und Ableitungsmittel (14) eingebaut sind;
- einen Träger (3), damit sich der Patient hinlegen kann, wobei der Träger zumindest teilweise, aber im Wesentlichen derart transparent oder farbig ist, dass er die von der Lichtkammer (10) kommende Lichtstrahlung durchlässt, wobei der Träger (3) oberhalb der Lichtkammer (10) angeordnet ist, sodass der auf dem Träger (3) gehaltene Patient von unten in Bezug auf seine Schwerpunktsrichtung bestrahlt wird, wodurch eine bessere Bestrahlung der Bereiche mit starker Bilirubinkonzentration, die dazu neigen, dem Schwerpunkt zu folgen, möglich ist; und wobei in der Vorrichtung die mindestens eine Lichtstrahlungsquelle Lampen (11, 12) aufweist und die Lichtkammer (10) eine Temperaturkontrollvorrichtung aufweist, **dadurch gekennzeichnet, dass**
- die Lampen ein Lichtspektrum mit einer Wellenlänge von 420 bis 650 nm mit einem deutlichen Anteil an blauem und grünem Licht aufweisen und die Bereitstellung einer Bestrahlungsstärke von über 3 mW pro cm² der Haut des Patienten ermöglichen; und das
- die Temperaturkontrollvorrichtung einen Thermostat (17) betätigt, der das Einschalten und Ausschalten der Lampen (11, 12) und die Aktivierung der Ventilatoren (7) steuert.

2. Vorrichtung nach Anspruch 1, **dadurch gekennzeichnet, dass** die Stärke der Bestrahlung des Patienten, die durch die Lichtstrahlung erzielt wird, über 5 mW pro cm² der Haut des Patienten beträgt.

3. Vorrichtung nach einem der Ansprüche 1 oder 2, **dadurch gekennzeichnet, dass** der Träger (3) aus einem Material hergestellt ist, das den Durchtritt von ultravioletter und infraroter Strahlung filtert oder sperrt.

4. Vorrichtung nach einem der vorangegangenen Ansprüche, **dadurch gekennzeichnet, dass** der Träger (3) eine Matratze umfasst.

5. Vorrichtung nach Anspruch 4, wobei die Matratze vom Geltyp ist.

6. Vorrichtung nach einem der vorangegangenen Ansprüche, **dadurch gekennzeichnet, dass** der Träger (3) eine Plexiglas^{®}-Platte umfasst oder aus einer solchen besteht.

7. Vorrichtung nach Anspruch 1, **dadurch gekennzeichnet, dass** die Lampen keinen Zünder aufweisen.

8. Vorrichtung nach einem der vorangegangenen Ansprüche, **dadurch gekennzeichnet, dass** die Lampen (11, 12) alternierend an einander gegenüberliegenden Seiten der Lichtkammer (10) angeordnet sind.

9. Vorrichtung nach einem der vorangegangenen Ansprüche, **dadurch gekennzeichnet, dass** sie eine oder mehrere Kuppeln (5) umfasst, die in Bezug auf den Träger (3) an der der Lichtkammer (10) gegenüberliegenden Seite angeordnet sind und mindestens eine der Lampen aufweisen.

10. Vorrichtung nach einem der vorangegangenen Ansprüche, **dadurch gekennzeichnet, dass** der Boden oder die Seitenwände der Lichtkammer (10) oder der Kuppeln (5) ein oder mehrere reflektierende Elemente aufweisen, die derart angeordnet sind, dass sie eine Beleuchtung des Patienten sicherstellen.

11. Inkubator oder geschlossener Brutkasten, in dem die Lichttherapievorrichtung nach einem der vorangegangenen Ansprüche eingebaut ist.
